# EUROPEAN PATENT APPLICATION

(11) **EP 4 413 977 A1**
(43) Date of publication of application: **14.08.2024**
(21) Application number: 22896097.7
(22) Date of filing: 17.11.2022
(51) Int. Cl.: A61K 9/16

(54) **METHOD FOR PREPARING MICROPARTICLES CONTAINING POORLY SOLUBLE DRUGS**

(30) Priority: 18.11.2021 KR 20210159539
(71) Applicant: Inventage Lab Inc., Gyeonggi-do 13403 (KR)
(72) Inventor: KIM, Ju Hee, Seongnam-si, Gyeonggi-do 13494 (KR)
(74) Representative: BCKIP Part mbB
(86) International application number: PCT/KR2022/018216
(87) International publication number: WO 2023/090899

(57) **Abstract**

The present invention relates to a method for producing microparticles containing a poorly soluble drug, and microparticles produced by the method. According to the method for preparing microparticles, it is possible to produce microparticles that are uniform and of good quality and have high encapsulation efficiency for the poorly soluble drug and low contents of residual organic solvents, by using at least two organic solvents.

## Description

### Technical Field

The present invention relates to a method for producing microparticles containing a poorly soluble drug.

### Background Art

In order to produce microparticles or sustained-release formulations containing a poorly soluble drug, the poorly soluble drug together with a biodegradable polymer is generally dissolved in an organic solvent and the solution is dispersed in a water phase, thus preparing an emulsion. For example, a sustained-release formulation containing a poorly soluble drug may be prepared through a process of preparing an oil-in-water (O/W) emulsion to form microparticles (also referred to as microspheres) containing the poorly soluble drug, and then removing the organic solvent from the emulsion. It is important that, in the process of preparing the emulsion to form microparticles, both the poorly soluble drug and the biodegradable polymer are well dissolved in the oil phase solution, and the poorly soluble drug is prevented from being lost into the water phase solution, thereby increasing the encapsulation efficiency of the poorly soluble drug in the biodegradable polymer, and in the process of removing the organic solvent after formation of the microparticles, the poorly soluble drug is prevented from being lost by being transferred to the water phase solution. This may vary depending on which organic solvent is selected, and thus the choice of solvent is critical to the method of producing microparticles containing a poorly soluble drug.

In the process of preparing the emulsion for the formation of microparticles or the production of a sustained-release formulation, dissolving the biodegradable polymer is as important as dissolving the drug component in the solvent. This is because the drug needs to be encapsulated in the biodegradable polymer in order to achieve sustained release of the drug. In general, as an organic solvent for dissolving the biodegradable polymer, for example, dichloromethane may be used. However, since some poorly soluble drugs exhibit relatively low solubility in dichloromethane, a large amount of the dichloromethane solvent needs to be used to dissolve the poorly soluble drug in the solvent. However, an increase in the amount of solvent used leads to an increase in the possibility for residual organic solvent to remain, as well as an increase in the possibility for the poorly soluble drug to be lost from the oil phase solution into the water phase solution in the process of producing microparticles. Accordingly, a problem arises in that the drug component in the microparticles is lost and the encapsulation efficiency of the drug in the biodegradable polymer is lowered.

In addition, methods for producing such microparticles include, for example, a method using a porous membrane, a microfluidic method using a microchannel, an emulsion method, or a spray-drying method.

In the method of producing the microparticles, the viscosity of the oil phase solution containing the poorly soluble drug and the biodegradable polymer plays an important role in the production process, and the choice of the type and content of organic solvent is important to control the viscosity of the oil phase solution. However, as described above, if the content of the organic solvent is increased to control viscosity, problems arise in that the possibility of loss of the poorly soluble drug increases and the possibility for residual organic solvent to remain increases.

In addition, the method of removing the solvent from the microparticles may vary depending on the nature of the solvent in the removal process, and thus the type of solvent used to prepare the oil phase solution may affect the encapsulation efficiency of the poorly soluble drug in the biodegradable polymer, the content of residual organic solvent, and the economic efficiency of the production method. Methods for removing a solvent from microparticles include a solvent evaporation method and a solvent extraction method.

The solvent evaporation method is a method of removing a volatile solvent with a relatively low boiling point by evaporating the solvent by heating to the boiling point of the solvent. This method has advantages over the solvent extraction method in that the process is easier and requires a shorter time. However, this method may have problems in that, depending on the characteristics of the drug corresponding to the active ingredient of the microparticles, the drug may be lost along with the solvent due to increased temperature and the encapsulation efficiency of the drug in the biodegradable polymer decreases.

The solvent extraction method is a method of removing a non-volatile solvent with a relatively high boiling point by diffusing the same from the microparticles to an external solvent by a concentration gradient. In order to prevent the drug from being extracted together with the solvent, solvent extraction is performed at a low temperature, and in order to efficiently perform extraction with a solvent such as benzyl alcohol, a co-solvent such as ethyl acetate or ethanol is added to the water phase solution outside the microparticles.

In an attempt to increase the solubility of poorly soluble drugs, a method of improving the solubility using benzyl alcohol as a solubilizing agent has been studied wherein the solvent extraction method is used. In other words, since benzyl alcohol has a high boiling point of 205.4°C, the solvent extraction method rather than the solvent evaporation method should be used to effectively remove benzyl alcohol. For example, an oil phase solution is prepared by dissolving a poorly soluble drug and a biodegradable polymer in dichloromethane and benzyl alcohol, and the oil phase solution is dispersed in a water phase to form microparticles, and then the benzyl alcohol is removed using ethyl acetate or ethanol as an extraction solvent.

However, in the solvent extraction method described above, extraction occurs based on a concentration gradient, and thus the solvent is removed in proportion to the extraction time.

Therefore, it takes a lot of work time to completely remove the residual organic solvent. If a solvent such as benzyl alcohol remains without being completely removed, the solvent may act as a solubilizer for the drug component, causing a burst effect in which the drug which needs to be released slowly is released all at once from the microparticles, deviating from the release mechanism from the biodegradable polymer, and it may be difficult to control drug release.

As such, the solvent extraction method has disadvantage over the solvent evaporation method in that the process is complicated and time-consuming.

Therefore, there is a need to develop an economic and efficient method for producing microparticles containing a poorly soluble drug, which may dissolve the poorly soluble drug easily, increase the encapsulation efficiency of the drug in the biodegradable polymer, increase the convenience of production because it is simple, and provide uniform microparticles.

### [Prior Art Documents]

### [Patent Documents]

(Patent Document 1) KR 10-2005-0093236 A1

### DISCLOSURE

### Technical Problem

An object of the present invention is to provide a method for producing microparticles containing a poorly soluble drug.

Another object of the present invention is to provide a method for producing microparticles, in which at least two organic solvents are used to dissolve the poorly soluble drug, and thus it is possible to easily produce microparticles that are uniform and of good quality and have high encapsulation efficiency for the poorly soluble drug and low contents of residual organic solvents.

Still another object of the present invention is to provide a method for producing microparticles, in which small amounts of organic solvents are used to dissolve a poorly soluble drug and a biodegradable polymer, so that the viscosity or density of the oil phase solution may be lowered, and when microparticles are produced using a microfluidic method, the laminar flow within the microchannel may be maintained, thus producing microparticles that are homogeneous and of good quality while having a high encapsulation efficiency for the poorly soluble drug.

### Technical Solution

To achieve the above objects, the present invention provides a method for producing microparticles containing a poorly soluble drug, the method comprising steps of: 1) preparing an oil phase solution by dissolving a poorly soluble drug and a biodegradable polymer in a mixed solvent comprising at least two organic solvents; 2) preparing a water phase solution by dissolving a surfactant in water; and 3) producing microparticles using the oil phase solution and the water phase solution.

The mixed solvent may comprise a first solvent and a co-solvent, wherein the first solvent may be dichloromethane.

The co-solvent may have a density of 1.3 g/cm³ or less, a polarity index of 3 or less, a boiling point of 50°C or lower, or a water solubility of 2²⁰ to 8²⁰ g/100 g water.

The first solvent and the co-solvent may be comprised at a weight ratio of 1:0.5 to 1: 10.

The poorly soluble drug may be naltrexone, donepezil, finasteride, aripiprazole, olanzapine, palonosetron, minocycline, memantine, alendronate, deoxycholate, risedronate, ibandronate, zoledronate, liraglutide, exenetide, lanreotide, octreotide, deslorelin, leuprorelin, goserelin, triptorelin, or dutasteride.

The poorly soluble drug and the mixed solvent in step 1) may be mixed together at a weight ratio of 1:7 to 1:30.

The poorly soluble drug and the biodegradable polymer in step 1) may be comprised at a weight ratio of 1:0.5 to 1:10.

The biodegradable polymer may be selected from the group consisting of polylactide, polylactic acid, polylactide-co-glycolide, polylactic-co-glycolic acid, polyphosphazine, polyiminocarbonate, polyphosphoester, polyanhydride, polyorthoester, polycaprolactone, polyhydroxyvalate, polyhydroxybutyrate, polyamino acid, and combinations thereof.

The surfactant may be selected from the group consisting of polyethylene glycol sorbitan monooleate, sorbitan oleate, sodium lauryl sulfate, polyvinyl alcohol (PVA), methylcellulose, polyvinylpyrrolidone, lecithin, gelatin, polyoxyethylene sorbitan fatty acid esters, polyoxyethylene castor oil derivatives, sodium stearate, ester amines, linear diamines, fatty amines, and combinations thereof.

The microparticles in step 3) may be produced using the oil phase solution and the water phase solution by an emulsion method, a porous membrane method, a spray-drying method, or a microfluidic method.

The method may further comprise a step of removing residual organic solvents from the microparticles produced in step 3).

The step of removing the residual organic solvents may comprise adding the microparticles containing the residual organic solvents to the water phase solution and performing a stirring process to remove the residual organic solvents.

The stirring process may comprise: a first stirring step which is performed at 200 to 400 rpm at 10°C to 20°C for 30 minutes to 2 hours; a second stirring step which is performed at 200 to 400 rpm at 25°C to 35°C for 30 minutes to 2 hours; and a third stirring step which is performed at 200 to 400 rpm at 45°C to 55°C for 30 minutes to 2 hours.

Microparticles containing a poorly soluble drug according to another embodiment of the present invention may be produced by the above-described production method.

The microparticles may have an encapsulation efficiency of 90% or more for the poorly soluble drug, a smooth surface, and a perfectly spherical shape.

### Advantageous Effects

According to the present invention, at least two organic solvents are used to dissolve a poorly soluble drug, and thus it is possible to easily produce microparticles that are uniform and of good quality and have high encapsulation efficiency for the poorly soluble drug and low contents of organic solvents.

In addition, small amounts of organic solvents are used to dissolve a poorly soluble drug and a biodegradable polymer, and thus the viscosity or density of the oil phase solution may be lowered, and when microparticles are produced using a microfluidic method, the laminar flow within the microchannel may be maintained, thus producing microparticles that are homogeneous and of good quality while having high encapsulation efficiency for the poorly soluble drug.

### Best Mode

The present invention provides a method for producing microparticles containing a poorly soluble drug, the method comprising steps of: 1) preparing an oil phase solution by dissolving a poorly soluble drug and a biodegradable polymer in a mixed solvent comprising at least two organic solvents; 2) preparing a water phase solution by dissolving a surfactant in water; and 3) producing microparticles using the oil phase solution and the water phase solution.

### Mode for Invention

Hereinafter, embodiments of the present invention will be described in detail so that they can be easily carried out by those skilled in the art. However, the present invention may be embodied in various different forms and is not limited to the embodiments described below.

In order for microparticles containing a poorly soluble drug to exhibit excellent therapeutic effects, the poorly soluble drug should be dissolved well and contained in the microparticles at a high rate, the contents of residual organic solvents unrelated to the efficacy of the drug should be low, and the microparticles should have a uniform size.

Poorly soluble drugs in salt form are soluble to some extent in water at room temperature (e.g., naltrexone hydrochloride, a poorly soluble drug, has a water solubility of 100 mg/mL at 25°C), but poorly soluble drugs in free base form are barely soluble in water and do not completely dissolve even in organic solvents, indicating poor solubility. Organic solvents for producing microparticles containing a poorly soluble drug in free base form and having excellent properties should prevent the poorly soluble drug from being lost into the water phase solution while being capable of dissolving the poorly soluble drug together with a biodegradable polymer, and should be able to be easily removed after production of the microparticles.

Therefore, it is important to select organic solvents with suitable properties, which satisfy these requirements.

In addition, the choice of solvents is also important in order to economically and efficiently produce microparticles of uniform size. For example, in the case of a microfluidic method, in order to produce microparticles of uniform size, the water phase and the oil phase in the microchannel should be maintained in a laminar flow state.

Even for the same fluid, the Reynolds number (Re) varies depending on the fluid's viscosity, density and flow rate in the microchannel, the length of the channel, etc. When the Reynolds number is 2,300 or less, a laminar flow is formed, and when the Reynolds number is 4,000 or more, a turbulent flow is formed. A turbulent flow applies a non-uniform force to the particles in the oil phase solution (i.e., dispersed phase) when the oil phase solution is introduced into a microchannel through which the water phase solution flows, and thus it may hinder the formation of oil phase particles of uniform size, thereby reducing the quality and production yield quality of the microparticles. Therefore, in order to form a laminar flow, the velocity of the fluid should be lowered or the viscosity and/or density of the oil phase solution should be lowered.

The method of lowering the velocity of the fluid has the advantage of being able to easily form a laminar flow by changing production conditions, but the lowering of the velocity may result in a decrease in productivity.

Therefore, it is needed to use a suitable solvent that may lower the viscosity or density of the oil phase solution, in order to maintain a laminar flow while ensuring productivity.

As another method capable of producing microparticles is a method of producing microparticles using a porous membrane. In this method, the viscosity of the oil phase solution is important because the oil phase solution should pass well through the pores of the membrane.

Even in the emulsion method which is another method, the viscosity of the oil phase solution is important because the dispersion of the oil phase solution by external energy becomes unfavorable if the viscosity is excessively high.

In addition, even in the case of the spray drying-method, it is important to use a highly volatile solvent, because microparticles are produced by dispersing droplets and volatilizing the solvent by air blowing. In the production of microparticles, the choice of a solvent is also important to lower solvent volatilization energy.

In the production of microparticles, when an excessive amount of a solvent is used to achieve sufficient viscosity and when a microfluidic method is used, it may be easy to maintain a laminar flow or produce microparticles, but it takes a lot of energy and time to remove the excessive amount of the solvent, and it is difficult to quickly remove the organic solvent from the oil phase particles (dispersed phase), thus increasing the possibility for the drug to be lost by being transferred to the water phase solution.

Accordingly, the present invention relates to a method for producing microparticles, which may overcome the above-described problems, increase the encapsulation efficiency for a poorly soluble drug, and efficiently remove residual organic solvents from microparticles.

Specifically, the method for producing microparticles containing a poorly soluble drug according to the present invention may comprise steps of: 1) preparing an oil phase solution by dissolving a poorly soluble drug and a biodegradable polymer in a mixed solvent comprising at least two organic solvents; 2) preparing a water phase solution by dissolving a surfactant in water; and 3) producing microparticles using the oil phase solution and the water phase solution.

The mixed solvent for dissolving the poorly soluble drug and the biodegradable polymer may comprise a first solvent and a co-solvent, wherein the first solvent may be dichloromethane.

In general, a solvent in which a drug and a biodegradable polymer are easily dissolved is generally used to produce microparticles using an organic solvent, wherein the solvent that is generally used may be dichloromethane.

However, even when the organic solvent such as dichloromethane is used, some poorly soluble drugs have low solubility. To solve this problem, the present invention is characterized in that a co-solvent is additionally used in addition to the first solvent to increase the solubility of the poorly soluble drugs and to facilitate removal of the organic solvents later.

The co-solvent may have a density of 1.3 g/cm³ or less, a polarity index of 3 or less, a boiling point of 50°C or lower, or a water solubility of 2²⁰ to 8²⁰ g/100 g water. Specifically, the co-solvent may have a density of 1.3 g/cm³ or less, 0.5 to 1.3 g/cm³, 0.5 to 1.0 g/cm³, or 0.6 to 0.9 g/cm³.

In addition, the co-solvent may have a polarity index of 3 or less, 1 to 3, or 2 to 3.

In addition, the co-solvent may have a boiling point of 50°C or lower, 30°C to 50°C, or 30°C to 40°C.

In addition, the co-solvent may have a water solubility of 2²⁰ to 8²⁰ g/100 g water, 3²⁰ to 8²⁰ g/100 g water, or 5²⁰ to 8²⁰ g/100 g water.

When a co-solvent that satisfies the above-described density, polarity index, boiling point or water solubility conditions is used in combination with the first solvent, it serves to help the first solvent and increase the solubility of the poorly soluble drug. In addition, when the co-solvent is used in the process of removing residual organic solvents from the produced microparticles, the co-solvent may prevent the drug from being lost into the water phase solution while being removed earlier than the first solvent dichloromethane, and it may increase the concentration or viscosity of the biodegradable polymer present in the oil phase solution and increase the bonding strength between the poorly soluble drug and the biodegradable polymer, thus increasing the encapsulation efficiency of the drug in the biodegradable polymer.

In addition, since the co-solvent has a boiling point of 50°C or lower, even when it is heated during the solvent removal process, it may not change the properties of the biodegradable polymer and may not change the release pattern of the microparticles. In addition, since the co-solvent has a low density, even when it is used in small amounts, it may lower the viscosity or density of the oil phase solution, thereby making it possible to produce microparticles with uniform and good quality.

The above-described co-solvent may specifically be a volatile organic solvent or a volatile non-polar organic solvent.

The volatile organic solvent may be selected from the group consisting of acetone, acetonitrile, benzene, butyl alcohol, carbon disulfide, carbon tetrachloride, chloroform, cyclohexane, 1,1-dichloroethane, dimethoxyethane, ethanol, diethyl ether, ethyl acetate, heptane, hexane, methanol, methyl acetate, methyl t-butyl ether, pentane, propyl alcohol, tetrahydrofuran, and combinations thereof.

In addition, the volatile non-polar organic solvent may be selected from the group consisting of cyclohexane, pentane, hexane, heptane, carbon tetrachloride, carbon disulfide, benzene, diethyl ether, methyl t-butyl ether, tetrahydrofuran, ethyl acetate, and methyl acetate, chloroform, and combinations thereof.

When the co-solvent is used as a mixed solvent with the first solvent and acts together with the first solvent dichloromethane, it may lower the viscosity of the oil phase solution while increasing rather than decreasing the solubility of the poorly soluble drug or the biodegradable polymer, even though the co-solvent itself does not easily dissolve the poorly soluble drug or the biodegradable polymer.

Also, as described above, the co-solvent may have the property of volatilizing or evaporating earlier than dichloromethane. In general, the transfer of a drug to a water phase solution occurs on the surfaces of microparticles that have not been completely dried, and as the internal viscosity increases and curing occurs while the organic solvent remaining in microparticles is removed, the reactivity with the water phase solution decreases, and thus the likelihood of drug transfer into the water phase solution is lowered.

Accordingly, when a co-solvent having the above-described characteristics is used, the co-solvent may prevent the drug from being lost into the water phase solution while being removed earlier than the first solvent dichloromethane, and it may increase the concentration or viscosity of the biodegradable polymer present in the oil phase solution and increase the bonding strength between the poorly soluble drug and the biodegradable polymer, thus increasing the encapsulation efficiency of the drug in the biodegradable polymer.

In order to produce microparticles with uniform and good quality, the viscosity or density of the oil phase solution containing microparticles, the biodegradable polymer and the organic solvents is important. Since the co-solvent has a lower density than the first solvent, it may lower the density of the oil phase solution even when used in small amounts. Thus, when microparticles are to be produced by a microfluidic method, the co-solvent makes it possible to produce microparticles with uniform and good quality by allowing the oil phase solution and the water phase solution in the microchannel to be maintained in a laminar flow state and enables easy removal of residual organic solvents.

The co-solvent may preferably be diethyl ether or pentane, but is not limited to the above example, and it is possible to use, without limitation, any co-solvent that satisfies the above-described co-solvent conditions, increases the solubility of the poorly soluble drug when used together with the first solvent, and has the property of volatilizing or evaporating earlier than the first solvent.

In step 1), the weight ratio between the poorly soluble solvent and the mixed solvent may be about 1:7 to about 1:30, about 1:7 to about 1:29, about 1:7 to about 1:28, about 1:7 to about 1:27, about 1:7 to about 1:26, about 1:7 to about 1:25, about 1:7 to about 1:24, about 1:7 to about 1:23, about 1:7 to about 1:22, about 1:7 to about 1:21, about 1:7 to about 1:20, about 1:7 to about 1:19, about 1:7 to about 1:18, about 1:7 to about 1:17, about 1:7 to about 1:16, or about 1:7 to about 1:15, without being limited thereto.

In step 1), the weight ratio between the poorly soluble drug and the biodegradable polymer may be about 1: 0.5 to about 1:10, about 1: 0.5 to about 1:9, about 1: 0.5 to about 1:8, about 1:0.5 to about 1:7, about 1:0.5 to about 1:6, or about 1:1 to about 1:5, without being limited thereto.

In step 1), the weight ratio between the co-solvent and the first solvent may be about 1: 0.5 to about 1:10, about 1: 0.5 to about 1:9, about 1: 0.5 to about 1:8, about 1:0.5 to about 1:7, or about 1:0.5 to about 1:6, without being limited thereto.

Preferably, the weight ratio between the poorly soluble drug and the mixed solvent may be 1:15 to 1:20, and the weight ratio between the co-solvent and the first solvent may be 1:0.5 to 1:6, without being limited to the above examples. The poorly soluble drug may be dissolved well within the above weight ratio range, and if the mixed solvent is used in an amount smaller than the lower limit of the above range, problems may arise in that the poorly soluble drug recrystallizes and precipitates, and the viscosity increases excessively, making filtration and production difficult. If the mixed solvent is used in excessively large amounts, there is no great problem in production, but the absolute amount of organic solvent used increases, and thus the poorly soluble drug may be lost into the water phase solution and it may be difficult to remove residual organic solvents.

The content of the biodegradable polymer in the mixed organic solvent may be, but is not limited to, about 5 to about 50 wt%, about 5 to about 40 wt%, about 5 to about 30 wt%, about 5 to about 20 wt%, about 5 to about 10 wt%, based on the amount of biodegradable polymer (e.g., polylactide-co-glycolide copolymer) used. The total amount of mixed organic solvent used may vary depending on the viscosity of the biodegradable polymer and the amount of poorly soluble drug used. If the amount of the poorly soluble drug is large or the viscosity of the biodegradable polymer is high, the overall concentration may be lowered by increasing the amount of mixed organic solvent used. However, when the biodegradable polymer is dissolved in the mixed organic solvent within the above-described range, convenience in producing the microparticles may be achieved, and residual organic solvents may also be easily removed.

The poorly soluble drug may be naltrexone, donepezil, finasteride, aripiprazole, olanzapine, palonosetron, minocycline, memantine, alendronate, deoxycholate, risedronate, ibandronate, zoledronate, liraglutide, exenetide, lanreotide, octreotide, deslorelin, leuprorelin, goserelin, triptorelin, or dutasteride.

Naltrexone may also be called N-cyclopropyl-methylnoroxymorphone, N-cyclopropylmethyl-14-hydroxydihydro-morphinone, 17-(cyclopropylmethyl)-4,5α-epoxy-3,14-dihydroxymorphinan-6-one, EN-1639A, or UM-792.

Naltrexone may be a compound represented by the following Formula:

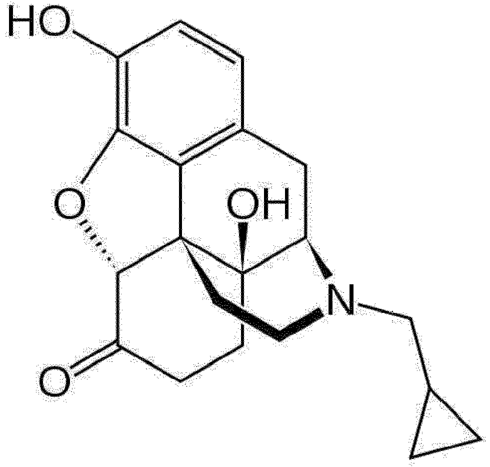

Donepezil may also be called 1-benzyl-4-[(5,6-dimethoxy-1-indanon-2-yl)methyl]piperidine.

Donepezil may be a compound represented by the following Formula:

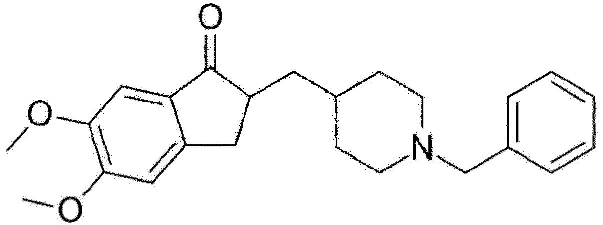

Finasteride may also be called N-(1,1-dimethylethyl)-3-oxo-(5α,17β)-4-azaandrost-1-ene-17-carboxamide.

Finasteride may be a compound represented by the following Formula:

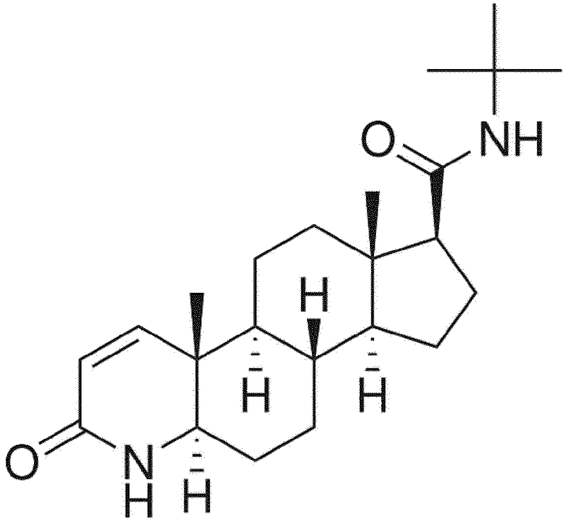

The poorly soluble drug of the present invention may be in the form of a solvate, stereoisomer, prodrug, metabolite (e.g., 6β-naltrexol), derivative (e.g., naloxone), free base, or combination thereof, of the poorly soluble drug.

The stereoisomer refers to isomers that have the same molecular formula and sequence of bonded atoms, but differ in the arrangement of their atoms in space. The solvate refers to a compound solvated in an organic or inorganic solvent. The solvate is, for example, a hydrate. The stereoisomer may be a diastereomer or enantiomer. The prodrug may be a compound that changes into a target compound *in vivo* after administration of the compound. The metabolite may be a compound produced through an *in vivo* metabolic process. The derivative refers to a compound obtained by replacing part of the structure of the poorly soluble drug with another atom or atomic group.

The biodegradable polymer may be selected from the group consisting of polylactide, polylactic acid, polylactide-co-glycolide, polylactic-co-glycolic acid, polyphosphazine, polyiminocarbonate, polyphosphoester, polyanhydride, polyorthoester, polycaprolactone, polyhydroxyvalate, polyhydroxybutyrate, polyamino acid, and combinations thereof, without being limited to the above examples.

In one example, the molar ratio of glycolide to lactide in polylactide-co-glycolide may be about 60:40 to about 90:10, about 60:40 to about 85:15, about 60:40 to about 80:20, about 60:40 to about 75:25, about 65:35 to about 90:10, about 70:30 to about 90:10, about 75:25 to about 90:10, about 65:35 to about 85:15, or about 70:30 to about 80:20, without being limited to the above examples. Preferably, the molar ratio of glycolide to lactide in polylactide-co-glycolide may be about 75:25.

The biodegradable polymer may comprise at least one polylactide and at least one polylactide-co-glycolide copolymer. In the present invention, the biodegradable polymer may comprise, for example, two polylactides, a combination of one polylactide and one polylactide-co-glycolide copolymer, two polylactide-co-glycolide copolymers, three polylactides, a combination of two polylactides and one polylactide-co-glycolide copolymer, a combination of one polylactide and two polylactide-co-glycolide copolymers, or the like. In particular, the biodegradable polymer may comprise a combination of one polylactide and one polylactide-co-glycolide copolymer, or two polylactide-co-glycolide copolymers, without being limited thereto.

The biodegradable polymer may comprise at least two polylactide-co-glycolide copolymers.

The water phase solution may contain water and a surfactant. Here, as the surfactant, any surfactant that may help the oil phase solution form stable microparticles may be used without limitation.

Specifically, the surfactant may be at least one selected from the group consisting of nonionic surfactants, anionic surfactants, cationic surfactants, and combinations thereof. For example, the surfactant may be at least one selected from the group consisting of polyethylene glycol sorbitan monooleate, sorbitan oleate, sodium lauryl sulfate, polyvinyl alcohol (PVA), methylcellulose, polyvinylpyrrolidone, lecithin, gelatin, polyoxyethylene sorbitan fatty acid esters, polyoxyethylene castor oil derivatives, sodium stearate, ester amines, linear diamines, fatty amines, and combinations thereof, without being limited thereto.

The content of the surfactant in the water phase solution may be 0.1 to 1.0% (w/v), 0.2 to 0.8% (w/v), 0.25 to 0.7% (w/v), 0.4 to 0.6% (w/v), 0.4 to 0.5% (w/v), 0.5 to 0.6% (w/v), 0.1 to 0.3% (w/v), 0.2 to 0.3% (w/v), or 0.25 to 0.3% (w/v), without being limited thereto. For example, the water phase solution containing the surfactant may be a 0.5% (w/v) PVA solution, without being limited to the above example.

The viscosity of the oil phase solution in step 1) may be in a range in which the viscosity (unit: centipoise (cP)) of the fluid allows the fluid in the microchannel to be maintained in a laminar flow state. The viscosity of the fluid may be measured with a Brookfield Model LVT viscometer using an LV 01 or LV 02 spindle at 80 to 100 rpm. The viscosity of the oil phase solution is measured at 25°C, and when the measurement is performed with a viscometer, a certain value of the viscosity is measured after the solution to be measured is stabilized. In general, it takes about 1 minute for the stabilization of the solution.

The oil phase solution of step 1) may have such a viscosity or density that it is maintained in a laminar flow state together with the water phase solution of step 2). Specifically, when the oil phase solution is introduced into the water phase solution flowing in the microchannel, the oil phase solution may have such a viscosity or density that the fluid in the microchannel is maintained in a laminar flow state. For example, the oil phase solution may have such a viscosity or density that the Reynolds number of the fluid flowing in the microchannel satisfies 2,300 or less.

The microparticles in step 3) may be produced using the oil phase solution and the water phase solution by an emulsion method, a porous membrane method, a spray-drying method, or a microfluidic method.

Specifically, the process of producing microparticles by the microfluidic method may comprise steps of: a) introducing the oil phase solution into a straight microchannel; b) introducing the water phase solution into a microchannel on one or either side; and c) collecting microparticles.

In step a), the oil phase solution is introduced into a straight microchannel and allowed to flow therethrough, and in step b), the water phase solution is introduced into a microchannel formed on one or either side so as to form an intersection point with the straight microchannel and allowed to flow therethrough. In other words, the oil phase solution may flow along the straight microchannel, and the water phase solution may flow along a microchannel, formed on one or either side of the straight microchannel so as to form an intersection point with the straight microchannel, and meet the flow of the oil phase solution.

In addition, in order to make the water phase solution forming an intersection point with the flow of the oil phase solution flow at a higher flow rate than the oil phase solution introduced into the straight microchannel, the water phase solution is allowed to flow under higher pressure conditions.

By varying the flow rates of the oil phase solution and the water phase solution and making the flow rate of the water phase solution higher than the flow rate of the oil phase solution as described above, the water solution with a relatively higher flow rate may compress the oil phase solution at the point where the flow of the oil phase solution meets the flow of the water phase solution meet, and at this time, the biodegradable polymer and poorly soluble drug in the oil phase solution may produce spherical microparticles due to the repulsive force between the oil phase solution and the water phase solution, and the spherical microparticles may have a structure in which the drug is evenly distributed in the spherical biodegradable polymer.

The method of producing microparticles by the microfluidic method is a method of forming microparticles of a certain size by introducing, into a microchannel, the oil phase solution in which the poorly soluble drug, the mixed organic solvent and the biodegradable polymer are dissolved, together with the water phase solution. This method may be a method of producing microparticles in the water phase solution. The micro-sized particles thus formed may be stabilized by the surfactant in the water phase solution, and as the organic solvent in the particles is evaporated or volatilized depending on the drying conditions, the organic solvent in the particles may be removed, thus forming microparticles.

The emulsion method may be a method comprising mixing the oil phase solution in which a poorly soluble drug, the mixed organic solvent and the biodegradable polymer are dissolved, and the water phase solution containing the surfactant, and then applying external energy (ultrasound, high-speed rotational force, etc.) to the mixture, causing the oil phase solution to form micro-sized particles in the water phase solution. As the organic solvent in the microparticles formed by the emulsion method is evaporated or volatilized depending on the drying conditions, the organic solvent in the particles may be removed, thus forming microparticles.

The porous membrane method is a method of producing microparticles by allowing the oil phase solution (dispersed phase), in which the poorly soluble drug, the mixed organic solvent and the biodegradable polymer are dissolved, to flow to one side of a porous membrane with micro-pores, and allowing the surfactant-containing water phase solution (continuous phase) to flow to the other side of the porous membrane to break the oil phase solution with the flow of the water phase solution.

The spray-drying method is a method of producing microparticles by spraying the oil phase solution, in which the poorly soluble drug, the mixed organic solvent and the biodegradable polymer are dissolved, in a spray dryer while blowing heated air, without using the water phase solution. In this method, micro-sized particles may be formed by atomizing the oil phase solution, and the organic solvent in the particles may be removed by evaporation or volatilization with heated air, thus forming microparticles.

Specific examples of the emulsion method and the spray-drying method are described, for example, in Koerner, J. (2019). Harnessing Dendritic Cells for Poly (D,L-lactide-co-glycolide) Microparticles (PLGA MS) - Mediated Anti-tumor Therapy. Frontiers, and Wang, Y (2016). Manufacturing Techniques and Surface Engineering of Polymer Based Nanoparticles for Targeted Drug Delivery to Cancer. Nanomaterials 6(2), 26, without being limited thereto.

Microparticles containing a poorly soluble drug according to another embodiment of the present invention are microparticles produced by the above-described production method.

The encapsulation efficiency for the poorly soluble drug in the microparticles may be about 90%, about 91%, about 92%, about 93%, about 94%, about 95%, about 96%, about 97%, about 98%, about 99%, or about 100%.

The microparticles may also be referred to as microspheres, and may refer to those particles which may contain the poorly soluble drug as an active ingredient therein.

The median particle size (D50) of the microparticles may be about 30 µm to about 65 µm, about 30 µm to about 60 µm, about 30 µm to about 55 µm, about 30 µm to about 50 µm, about 35 µm to about 65 µm, about 40 µm to about 65 µm, about 45 µm to about 65 µm, about 35 µm to about 60 µm, about 40 µm to about 55 µm, or about 45 µm to about 50 µm.

In one embodiment, the microparticles may have a particle size distribution in the range of the median particle size ± 5 µm, ± 7 µm, ± 10 µm, ± 12 µm, or ± 15 µm.

In addition, based on the total weight of the microparticles, at least 60 wt%, at least 65 wt%, at least 70 wt%, at least 75 wt%, at least 80 wt%, at least 85 wt%, at least 90 wt%, at least 95 wt%, or at least 99 wt% of microparticles may be within this particle size distribution range.

### [Experimental Example 1] Comparison of solubility according to combination of dichloromethane and diethyl ether

To compare the solubility of a poorly soluble drug according to the types and combination of solvents, 0.5 g of naltrexone in free base form (manufactured by Mallinckrodt; the same applies hereinafter), a poorly soluble drug, and 1.0 g of a biodegradable polymer (manufactured by Corbion; PDLG7504 (ester type); the same applies hereinafter) were mixed and dissolved in 7.0 g of organic solvent(s) as shown in Table 1 below, and whether naltrexone and the biodegradable polymer were dissolved at room temperature was visually observed. At this time, a transparent state in which no crystals or particles were visible to the naked eye was determined to be a completely dissolved state.

**[Table 1]**

| | Comparative Example 1 | Comparative Example 2 | Example 1 | Example 2 |
|---|---|---|---|---|
| Naltrexone | 0.5 g | 0.5 g | 0.5 g | 0.5 g |
| 75/25 DL-lactide/glycolide copolymer | 1.0 g | 1.0 g | 1.0 g | 1.0 g |
| Dichloromethane | 7.0 g | - | 5.0 g | 4.0 g |
| Diethyl ether | - | 7.0 g | 2.0 g | 3.0 g |
| Whether naltrexone and biodegradable polymer were dissolved | Recrystallized | Neither naltrexone nor biodegradable polymer was dissolved | Completely dissolved | Completely dissolved |

If dichloromethane is used in excessive amounts, it can dissolve naltrexone, but the above-described problems such as residual organic solvents may occur due to an increase in the total amount of solvents used. In order to avoid these problems, dichloromethane could be used alone in the smallest possible amount to dissolve naltrexone as in Comparative Example 1. However, in this case, a recrystallization phenomenon occurred in which naltrexone precipitated. It is believed that naltrexone precipitated as crystals from the saturated solution due to changes in pressure caused by compressed air and volatilization of the solvent when the oil phase solution was sprayed through the module together with the water phase solution. In addition, in Comparative Example 2, in which diethyl ether was used alone as the organic solvent, neither naltrexone nor the biodegradable polymer was dissolved at all. In contrast, in Examples 1 and 2, in which the co-solvent diethyl ether was mixed with the first solvent dichloromethane, both naltrexone and the biodegradable polymer were completely dissolved, the solubility became much higher than when each of the solvents was used alone, and no recrystallization phenomenon occurred.

This suggests that the use of diethyl ether, which has poor ability to dissolve naltrexone and the biodegradable polymer, as a co-solvent, affected the arrangement between the first solvent dichloromethane and naltrexone molecules, thereby increasing the solubility of naltrexone and the biodegradable polymer.

From the above experimental results, it can be seen that, when dichloromethane or diethyl ether was used alone, neither naltrexone nor the biodegradable polymer was dissolved, but when diethyl ether as a co-solvent was used in combination with dichloromethane, the solubility of naltrexone and the biodegradable polymer was increased.

As described above, it was confirmed that when dichloromethane and diethyl ether were used in combination, they could completely dissolve naltrexone and the biodegradable polymer even when the solvents were used in small amounts.

Accordingly, when microparticles are produced using the mixed solvent as in Examples 1 and 2, the loss of naltrexone can be reduced, increasing the encapsulation efficiency for naltrexone, and the amount of organic solvent used can be reduced, enabling the effective removal of residual organic solvents.

Thereafter, additional experiments were conducted to validate this effect.

### [Experimental Example 2] Experiment for comparing encapsulation efficiency for naltrexone microparticles, residual organic solvent, and dissolution according to solvent

### (1) Production of microparticles containing naltrexone

Microparticles for use in the experiment were produced as follows, and the contents of the components used in the production of the microparticles are summarized in Table 2 below.

### [Example 3]

0.5 g of naltrexone in free base form and 1.0 g of a DL-lactide/glycolide copolymer were mixed and dissolved in 10.0 g of dichloromethane and 2.3 g of diethyl ether. The resulting oil phase solution was applied to each microchannel to produce microparticles at the intersection between the oil phase solution and the water phase solution, and the microparticles were collected in the water phase solution (10°C). The water phase solution was a 0.5% (w/v) PVA solution (0.5% (v/v) PVA in water).

The produced microparticles were stirred at 10°C for 1 hour, at 30°C for 1 hour, and then at 50°C for 1 hour to remove the organic solvents. The produced microparticles were sieved and then freeze-dried, thereby producing dried microparticles.

### [Example 4]

Microparticles were produced in the same manner as in Example 3, except that 8.0 g of dichloromethane and 2.0 g of diethyl ether were used.

### [Comparative Example 3]

Microparticles were produced in the same manner as in Example 3, except that no diethyl ether was used and 8.0 g of dichloromethane was used.

### [Comparative Example 4]

Microparticles were produced in the same manner as in Example 3, except that no diethyl ether was used and 12.3 g of dichloromethane was used.

**[Table 2]**

| | Comparative Example 3 | Comparative Example 4 | Example 3 | Example 4 |
|---|---|---|---|---|
| Naltrexone | 0.5 g | 0.5 g | 0.5 g | 0.5 g |
| 75/25 DL-lactide/glycolide copolymer | 1.0 g | 1.0 g | 1.0 g | 1.0 g |
| Dichloromethane | 8.0 g | 12.3 g | 10.0 g | 8.0 g |
| Diethyl ether | - | - | 2.3 g | 2.0 g |

### (2) Comparison of encapsulation efficiency, residual organic solvent, and precipitation according to use of co-solvent and amount of organic solvent

For the microparticles produced in Experimental Example 2(1), solubility, encapsulation efficiency, and residual organic solvent were assessed. Whether naltrexone and the biodegradable polymer were dissolved was checked visually based on whether precipitation occurred, and the encapsulation efficiency was measured using high-performance liquid chromatography (HPLC). Residual organic solvents were analyzed through gas chromatogram (GC). The results are shown in Table 3 below.

**[Table 3]**

| | Encapsulation efficiency (%) | Residual organic solvent (ppm) | Remarks |
|---|---|---|---|
| Comparative Example 3 | - | - | Precipitated |
| Comparative Example 4 | 84.67 | Dichloromethane: 1,764.2 | - |
| Example 3 | 90.97 | Dichloromethane: 806.9; | - |
| | | diethyl ether: 322.9 | |
| Example 4 | 93.08 | Dichloromethane: 1,000.5; | - |
| | | diethyl ether: 289.8 | |

As a result of comparing Comparative Example 3 and Comparative Example 4, it was confirmed that, if the amount of dichloromethane was insufficient, naltrexone dissolved in the organic solvent was precipitated due to recrystallization during the process of preparing the oil phase solution (because naltrexone is poorly soluble in dichloromethane), and thus microparticles could not be produced. As a result of comparing Comparative Example 4 with Examples 3 and 4, it was confirmed that, when diethyl ether was used as a co-solvent, the encapsulation efficiency increased by about 6% compared to when dichloromethane was used alone, and in this case, the amount of the residual organic solvent dichloromethane was reduced, and the total amount of residual organic solvents was also reduced.

From the above results, it can be seen that the effect of increasing the encapsulation efficiency was obtained by using dichloromethane in combination with diethyl ether, which has a boiling point lower than that of dichloromethane. This is believed to be because the solvent diethyl ether volatilized at a lower temperature than dichloromethane, and thus the concentrations of naltrexone and the biodegradable polymer in the microparticles increased, increasing the viscosity of the oil phase solution present in the microparticles, thereby increasing the bonding strength between naltrexone and the biodegradable polymer.

[Experimental Example 3] Experiment for comparing encapsulation efficiency for naltrexone microparticles, residual organic solvent, and dissolution according to mixing ratio of solvents

### (1) Production of microparticles containing naltrexone

Microparticles for use in the experiment were produced as follows, and the contents of the components used in the production of the microparticles are summarized in Table 4 below.

### [Example 5]

0.5 g of naltrexone and 1.0 g of a DL-lactide/glycolide copolymer were mixed and dissolved in 6.0 g of dichloromethane and 2.0 g of diethyl ether. The resulting oil phase solution was applied to each microchannel to produce microparticles at the intersection between the oil phase solution and the water phase solution, and the microparticles were collected in the water phase solution (10°C). The water phase solution was a 0.5% (w/v) PVA solution. The produced microparticles were stirred at 10°C for 1 hour, at 30°C for 1 hour, and then at 50°C for 1 hour to remove the organic solvents. The produced microparticles were sieved and then freeze-dried, thereby producing dried microparticles.

### [Example 6]

Microparticles were produced in the same manner as in Example 5, except that microparticles were stirred at 10°C for 1.5 hours, at 30°C for 1.5 hours, and then at 50°C for 1.5 hours to remove the organic solvents.

### [Example 7]

Microparticles were produced in the same manner as in Example 5, except that 3.0 g of diethyl ether was used.

**[Table 4]**

| | Example 5 | Example 6 | Example 7 |
|---|---|---|---|
| Naltrexone | 0.5 g | 0.5 g | 0.5 g |
| 75/25 DL-lactide/glycolide copolymer | 1.0 g | 1.0 g | 1.0 g |
| Dichloromethane | 6.0 g | 6.0 g | 6.0 g |
| Diethyl ether | 2.0 g | 2.0 g | 3.0 g |

### (2) Comparison of encapsulation efficiency for naltrexone microparticles, residual organic solvent, and dissolution according to mixing ratio of solvents

**[Table 5]**

| | Encapsulation efficiency (%) | Residual organic solvent (ppm) | Remark |
|---|---|---|---|
| Example 5 | 98.97 | Dichloromethane: 1,360.0; | - |
| | | diethyl ether: 264.2 | |
| Example 6 | 90.70 | Dichloromethane: 1,827.7; | - |
| | | diethyl ether: 427.3 | |
| Example 7 | 101.19 | Dichloromethane: 1,207.2; | - |
| | | diethyl ether: 344.9 | |

As a result of comparing Example 5 and Example 6, it could be confirmed that, as the stirring time to remove the organic solvents increased, the encapsulation efficiency decreased rather than increased. In addition, it was confirmed that an increase in the stirring time to remove the organic solvents led to no significant change in the amount of residual organic solvent.

As a result of comparing Example 5 and Example 7, it was confirmed that the ratio between dichloromethane and diethyl ether used as the organic solvents affected the encapsulation efficiency. It can be seen that, as the ratio of diethyl ether to dichloromethane increased, the encapsulation efficiency for naltrexone in the microparticles increased.

[Experimental Example 4] Experiment using various kinds of organic solvent as co-solvent

The encapsulation efficiency of microparticles and the content of residual organic content upon the use of co-solvents other than diethyl ether were evaluated. Microparticles were produced in the same manner as in Example 3 of Experimental Example 2. The properties of usable co-solvents including diethyl ether are summarized in Table 6 below, and examples using various co-solvents are summarized in Table 7 below.

**[Table 6]**

| Solvent | Boiling point (°C) | Relative polarity | Water solubility (g/mL) | Vapor pressure (20→, hPa) |
|---|---|---|---|---|
| Methylene chloride | 39.8 | 0.309 | 1.32 | 475 |
| Diethyl ether | 34.6 | 0.117 | 7.5 | 587 |
| Pentane | 36.1 | - | 0.0039 | 573 |

### (1) Production of microparticles containing donepezil

### [Example 8]

0.98264 g of donepezil, 1.0 g of a DL-lactide/glycolide copolymer, and 3.0 g of a lactide copolymer were mixed and dissolved in 22.667 g of dichloromethane. The resulting oil phase solution was applied to each microchannel to produce microparticles at the intersection between the oil phase solution and the water phase solution, and the microparticles were collected in the water phase solution (10°C). The water phase solution was a 0.25% (w/v) PVA solution. The produced microparticles were stirred at 10°C for 1 hour, at 30°C for 1 hour, and then at 40°C for 3 hours to remove the organic solvent. The produced microparticles were sieved and then freeze-dried, thereby producing dried microparticles.

### [Example 9]

0.98264 g of donepezil, 1.0 g of a DL-lactide/glycolide copolymer, and 3.0 g of a lactide copolymer were mixed and dissolved in 6.0 g of dichloromethane and 4.5 g of diethyl ether. Subsequent procedures were performed in the same manner as in Example 8, thereby producing microparticles.

### [Example 10]

0.98264 g of donepezil, 1.0 g of a DL-lactide/glycolide copolymer, and 3.0 g of a lactide copolymer were mixed and dissolved in 8.0 g of dichloromethane and 3.0 g of pentane. Subsequent procedures were performed in the same manner as in Example 8, thereby producing microparticles.

### [Example 11]

0.98264 g of donepezil, 1.0 g of a DL-lactide/glycolide copolymer, and 3.0 g of a lactide copolymer were mixed and dissolved in 6.0 g of dichloromethane and 4.5 g of methyl-t-butyl ether. Subsequent procedures were performed in the same manner as in Example 8, thereby producing microparticles.

**[Table 7]**

| | Example 8 | Example 9 | Example 10 | Example 11 |
|---|---|---|---|---|
| Donepezil | 0.98264 g | 0.98264 g | 0.98264 g | 0.98264 g |
| 75/25 DL-DL-lactide/glycolide copolymer | 1.0 g | 1.0 g | 1.0 g | 1.0 g |
| Lactide copolymer | 3.0 g | 3.0 g | 3.0 g | 3.0 g |
| Dichloromethane | 22.667 g | 6.0 g | 8.0 g | 6.0 g |
| Diethyl ether | - | 4.5 g | - | - |
| Pentane | - | - | 3.0 g | - |
| Methyl-t-butyl ether | - | - | - | 4.5 g |

### (2) Comparison of encapsulation rate for donepezil microparticles and residual organic solvent according to kind of co-solvent

**[Table 8]**

| | Encapsulation efficiency (%) | Residual organic solvent (ppm) | Remarks |
|---|---|---|---|
| Example 8 | 105.95 | Dichloromethane: 3,744.1 | - |
| Example 9 | 106.38 | Dichloromethane: 440.4; | - |
| | | diethyl ether: 4,050.4 | |
| Example 10 | 88.83 | Dichloromethane: 435.4; | - |
| | | pentane: 25,537.9 | |
| Example 11 | 82.04 | Dichloromethane: 42.6; | Porous |
| | | methyl-t-butyl ether: 7,537.7 | |

As a result of comparing Example 8 and Example 9, it was confirmed that, in Example 9 in which the amount of dichloromethane used was reduced due to the use of diethyl ether as a co-solvent and drying was performed under the same conditions to remove dichloromethane, the content of dichloromethane as residual solvent was lower.

In addition, in the case of Example 10, the residual amount of pentane used as a co-solvent was measured to be very high. This is believed to be because the pentane could not be removed to the outside through water, as pentane has poor water solubility even though having a lower boiling point than diethyl ether.

In the case of Example 11, methyl-t-butyl ether as residual solvent appeared to be removed because it had a higher water solubility than pentane even though having a higher boiling point than pentane, but the solvent was not sufficiently removed because the boiling point thereof was higher than the final drying temperature. In addition, it could be seen that the microparticles had a non-smooth surface and were porous.

### [Experimental Example 5] Evaluation of increase in polymer proportion in oil phase solution by use of co-solvent

When a co-solvent is used in the preparation of an oil phase solution, the viscosity is lowered and a biodegradable polymer may be dissolved at a higher concentration than when dichloromethane is used alone as a solvent, making microparticle production possible.

### (1) Production of microparticles containing donepezil

**[Table 9]**

| | Example 8 | Example 12 | Example 13 | Example 14 | Example 15 | Example 16 |
|---|---|---|---|---|---|---|
| Donepezil | 0.98264 g | - | 0.98264 g | 0.98264 g | 0.98264 g | 0.98264 g |
| 75/25 DL-lactide/glycolide copolymer | 1.0 g | 1.0 g | 1.0 g | 1.0 g | 1.0 g | 1.0 g |
| Lactide copolymer | 3.0 g | 3.0 g | 3.0 g | 3.0 g | 3.0 g | 3.0 g |
| Dichloromethane | 22.667 g | 22.667 g | 6.0 g | 10.5 g | 6.0 g | 4.0 g |
| Diethyl ether | - | - | - | - | 4.5 g | 3.0 g |

### [Example 12]

1.0 g of a DL-lactide/glycolide copolymer and 3.0 g of a lactide copolymer were mixed and dissolved in 22.667 g of dichloromethane. Subsequent procedures were performed in the same manner as in Example 8, thereby producing microparticles.

### [Example 13]

0.98264 g of donepezil, 1.0 g of a DL-lactide/glycolide copolymer and 3.0 g of a lactide copolymer were mixed and dissolved in 6.0 g of dichloromethane. Subsequent procedures were performed in the same manner as in Example 8.

### [Example 14]

0.98264 g of donepezil, 1.0 g of a DL-lactide/glycolide copolymer and 3.0 g of a lactide copolymer were mixed and dissolved in 10.5 g of dichloromethane. Subsequent procedures were performed in the same manner as in Example 8.

### [Example 15]

0.98264 g of donepezil, 1.0 g of a DL-lactide/glycolide copolymer and 3.0 g of a lactide copolymer were mixed and dissolved in 6.0 g of dichloromethane and 4.5 g of diethyl ether. Subsequent procedures were performed in the same manner as in Example 8.

### [Example 16]

0.98264 g of donepezil, 1.0 g of a DL-lactide/glycolide copolymer and 3.0 g of a lactide copolymer were mixed and dissolved in 4.0 g of dichloromethane and 3.0 g of diethyl ether. Subsequent procedures were performed in the same manner as in Example 8.

### (2) Comparison of viscosity of oil phase solution according to ratio of co-solvent and checking of whether microparticle production is possible

**[Table 10]**

| | Solid content (%) | Viscosity (cp) | Whether production is possible |
|---|---|---|---|
| Example 8 | 18.02 | 10.9 | Possible |
| Example 12 | 15.00 | 11.0 | Possible |
| Example 13 | 45.37 | 399.9 | Impossible |
| Example 14 | 32.18 | 53.4 | Impossible |
| Example 15 | 32.18 | 28.6 | Possible |
| Example 16 | 41.58 | 109.0 | Possible |

The viscosity of Example 12, which is a placebo solution in which the active ingredient was not dissolved, was similar to that of Example 8, indicating that the active ingredient did not significantly affect the viscosity.

In addition, it could be confirmed that, in the case of Examples 13 and 14, in which dichloromethane was used alone as a solvent and the oil phase solution had a high solid content of more than 30%, microparticle production was impossible.

However, it could be confirmed that, in the case of Examples 15 and 16, in which the co-solvent diethyl ether was used, even though the solid content was more than 30%, the viscosity was lower than that in the dichloromethane single solvent group with the same solid content, and microparticle production was possible without problems.

The present inventors evaluated how uniform microparticles were produced depending on the viscosity or density of the oil phase solution for producing microparticles. In addition, the present inventors evaluated how much the production time of microparticles could be shortened and how efficiently residual organic solvents could be removed, depending on the viscosity or density of the oil phase solution for producing microparticles, thus evaluating whether the production of microparticles could be easily performed.

From the above description, it will be understood by those skilled in the art to which the present invention pertains that the present invention may be embodied in other specific forms without departing from the technical spirit or essential characteristics of the present invention. Therefore, the embodiments described above are considered to be illustrative in all respects and not restrictive. The scope of the present invention is defined by the appended claims rather than the detailed description, and it should be understood that all modifications and variations conceived from the meaning and scope of the claims and equivalents thereto are included within the scope of the present invention.

### Industrial Applicability

The present invention relates to a method for producing microparticles containing a poorly soluble drug.

The present invention was supported by the following national research and development project.
[Project Serial Number] 1465031634
[Grant Number] HI20C0936
[Government Department] The Ministry of Health and Welfare
[Project Management Agency] Korea Health Industry Development Institute
[Research Project Name] R&D linked to biohealth investment infrastructure
[Research Task Name] Development of long-acting injectable formulation for treatment of opioid and alcohol dependence using controlled and optimized production technology
[Contribution Rate] 1/1
[Agency Carrying Out Project] Inventage Lab Inc.
[Research Period] January 01, 2022 to December 31, 2022

## Claims

1. A method for producing microparticles containing a poorly soluble drug, the method comprising steps of:
1) preparing an oil phase solution by dissolving a poorly soluble drug and a biodegradable polymer in a mixed solvent comprising at least two organic solvents;
2) preparing a water phase solution by dissolving a surfactant in water; and
3) producing microparticles using the oil phase solution and the water phase solution.

2. The method according to claim 1, wherein the mixed solvent comprises a first solvent and a co-solvent, wherein the first solvent is dichloromethane.

3. The method according to claim 2, wherein the co-solvent has a density of 1.3 g/cm³ or less.

4. The method according to claim 2, wherein the co-solvent has a polarity index of 3 or less.

5. The method according to claim 2, wherein the co-solvent has a boiling point of 50°C or lower

6. The method according to claim 2, wherein the co-solvent has a water solubility of 2²⁰ to 8²⁰ g/100 g water.

7. The method according to claim 2, wherein the first solvent and the co-solvent are comprised at a weight ratio of 1:0.5 to 1: 10.

8. The method according to claim 1, wherein the poorly soluble drug is naltrexone, donepezil, finasteride, aripiprazole, olanzapine, palonosetron, minocycline, memantine, alendronate, deoxycholate, risedronate, ibandronate, zoledronate, liraglutide, exenetide, lanreotide, octreotide, deslorelin, leuprorelin, goserelin, triptorelin, or dutasteride.

9. The method according to claim 1, wherein the poorly soluble drug and mixed solvent in step 1) are mixed together at a weight ratio of 1:7 to 1:30.

10. The method according to claim 1, wherein the poorly soluble drug and biodegradable polymer in step 1) are comprised at a weight ratio of 1:0.5 to 1:10.

11. The method according to claim 1, wherein the biodegradable polymer is selected from the group consisting of polylactide, polylactic acid, polylactide-co-glycolide, polylactic-co-glycolic acid, polyphosphazine, polyiminocarbonate, polyphosphoester, polyanhydride, polyorthoester, polycaprolactone, polyhydroxyvalate, polyhydroxybutyrate, polyamino acid, and combinations thereof.

12. The method according to claim 1, wherein the surfactant is selected from the group consisting of polyethylene glycol sorbitan monooleate, sorbitan oleate, sodium lauryl sulfate, polyvinyl alcohol (PVA), methylcellulose, polyvinylpyrrolidone, lecithin, gelatin, polyoxyethylene sorbitan fatty acid esters, polyoxyethylene castor oil derivatives, sodium stearate, ester amines, linear diamines, fatty amines, and combinations thereof.

13. The method according to claim 1, wherein the microparticles in step 3) are produced using the oil phase solution and the water phase solution by an emulsion method, a porous membrane method, a spray-drying method, or a microfluidic method.

14. The method according to claim 1, further comprising a step of removing residual organic solvents from the microparticles produced in step 3).

15. The method according to claim 14, wherein the step of removing the residual organic solvents comprises adding the microparticles containing the residual organic solvents to the water phase solution and performing a stirring process to remove the residual organic solvents.

16. The method according to claim 15, wherein the stirring process comprises:
a first stirring step which is performed at 200 to 400 rpm at 10°C to 20°C for 30 minutes to 2 hours;
a second stirring step which is performed at 200 to 400 rpm at 25°C to 35°C for 30 minutes to 2 hours; and
a third stirring step which is performed at 200 to 400 rpm at 45°C to 55°C for 30 minutes to 2 hours.

17. Microparticles containing a poorly soluble drug, produced by the method for producing microparticles according to any one of claims 1 to 16.

18. The microparticles according to claim 17, which have an encapsulation efficiency of 90% or more for the poorly soluble drug, a smooth surface, and a perfectly spherical shape.
